# EUROPEAN PATENT SPECIFICATION

(11) **EP 0 964 697 B1**
(45) Date of publication and mention of the grant of the patent: **19.03.2003**
(21) Application number: 97948102.5
(22) Date of filing: 12.12.1997
(51) Int. Cl.: A61K 39/00, A61K 45/00, A61K 48/00

(54) **THERAPEUTIC APPLICATIONS OF ANTIGENS OR EPITOPES ASSOCIATED WITH IMPAIRED CELLULAR PEPTIDE PROCESSING, E.G. EXPRESSED ON RMA-S CELLS TRANSFECTED WITH A B7-1 GENE**
THERAPEUTISCHE ANWENDUNGEN VON ANTIGENEN ODER EPITOPEN ASSOZIERT MIT UNVOLLSTÄNDIGER ZELLULÄRER PEPTIDPROZESIERUNG , Z.B.: EXPRIMIERT IN RMA-S ZELLEN TRANSFIZIERT MIT B7-1 GEN
APPLICATIONS THERAPEUTIQUES D'ANTIGENES OU D'EPITOPES ASSOCIES A UN TRAITEMENT PEPTIDIQUE CELLULAIRE ALTERE, EXPRIMES PAR EXEMPLE SUR DES CELLULES RMA-S TRANSFECTEES AVEC UN GENE B7-1

(30) Priority: 12.12.1996 SE 9604581
(43) Date of publication of application: 22.12.1999
(73) Proprietor: Accuro Immunology AB, 11124 Stockholm (SE)
(72) Inventor: WOLPERT, Elisabeth, S-113 24 Stockholm (SE); KÄRRE, Klas, S-131 47 Nacka (SE); PETTERSSON, Max, S-113 57 Stockholm (SE); SANDBERG, Johan, S-171 38 Solna (SE)
(74) Representative: HOFFMANN - EITLE
(86) International application number: SE9702094
(87) International publication number: WO98025645

(56) References cited:
- EP-A- 0 600 591
- WO-A-93/24525
- WO-A-95/15384
- WO-A-96/09380
- WO-A-97/07128
- CH-A- 647 152
- DE-A- 2 024 458
- JOURNAL OF IMMUNOLOGY, Volume 156, 1996, S.K. NAIR et al., "Cells Treated with TAP-2 Antisense Oligonucleotides are Potent Antigen-Presenting Cells In Vitro and In Vivo", pages 1772-1780.
- DIALOG INFORMATION SERVICES, File 159, Cancerlit, Dialog Accession No. 01240395, Cancerlit Accession No. 96614957, MAYORDOMO J.I. et al.; & PROC. ANNU. MEET. AM. SOC. CLIN. ONCOL., 14:A1809, 1995.
- EUR. J. IMMUNOL., Volume 23, 1993, L. FRANKSSON et al., "Immunization Against Tumor and Minor Histocompatibility Antigens by Eluted Cellular Peptides Loaded on Antigen Processing Defective Cells", pages 2606-2613.
- JOURNAL OF IMMUNOLOGY, Volume 154, No. 7, 1995, TIANMIN LIU et al., "Heat-Inactivated Sendai Virus Can Enter Multiple MHC Class I Processing Pathways and Generate Cytotoxic T Lymphocyte Responses In Vivo", pages 3147-3155.
- SCAND. J. IMMUNOL., Volume 42, 1995, X. ZHOU et al., "Characterization of TAP-Independent and Brefeldin A-Resistant Presentation of Sendai Virus Antigen to CD8+ Cytotoxic T Lymphocytes", pages 66-75.
- PROC. NATL. ACAD. SCI. U.S.A., Volume 90, November 1993, R.A. HENDERSSON et al., "Direct identification of an Endogenous Peptide Recognized by Multiple HLA-A2.1-Specific Cytotoxic T Cells", pages 10275-10297
- BELLONE ET AL. IMMUNOLOGY TODAY vol. 20, no. 10, 1999, pages 457 - 462

## Description

The present invention relates to use of substances that can induce expression of antigens or epitopes associated with impaired cellular peptide processing, especially MHC class I dependent. for preparation of pharmaceuticals, pharmaceutical compositions or vaccines, that stimulate specific T cell mediated immune responses against cancer and virus infected cells. It also relates to use of antigens or epitopes associated with impaired cellular peptide processing, especially MHC class I dependent, or part thereof, for the same purpose. It also relates to mammalian cells that have been manipulated to express antigens or epitopes associated with impaired cellular peptide processing, especially MHC class I dependent, and to lymphoid cells activated against such MHC class I dependent structures for the same purpose. Furthermore, processes for such manipulation of mammalian cells and for treating human beings as well as kits for use in such manipulations are provided. The present invention also relates to use of molecules including T-cell receptors or part thereof directed against antigens or epitopes associated with impaired cellular peptide processing, especially MHC class I dependent, for preparation of pharmaceuticals, pharmaceutical compositions or vaccines. The ultimate purpose of the products or processes above is the treatment, prevention and diagnosis of cancers and virus infections.

### Background of the invention

The immune system recognizes material foreign to the body (so called antigen) and eliminates this material. An important part of the immune system is composed of CD8⁺ cytotoxic T cells or T-lymphocytes (CTL), which recognize foreign and sick cells, e.g. in virus infections or transplantation, and kill them. T cells recognize antigen via a T cell receptor on the surfaces thereof. The T cell receptor recognizes a cell surface molecule MHC (major histocompatibility complex) (HLA (human leucocyte antigen) in human beings), to which a peptide is attached. MHC class I molecules are expressed on all nucleated cells, they preferentially present endogenous cellular peptides. MHC class II molecules are preferentially expressed on professional antigen presenting cells and preferentially present peptides from extracellular antigens. The recognition structure on a target cell for T-cells, e.g. a peptide bound to an MHC molecule, is called an epitope. An epitope can often be part of a larger antigen, e.g. a protein.

The production and display of MHC class I complexes occurs through a peptide processing machinery within cells, whether these are normal, virus infected or transformed to cancer cells. Cells use proteasomes to degrade cytoplasmic proteins into short peptides (1). Some of these peptides are transported from the nucleus or cytoplasm to the endoplasmic reticulum (ER) or to the Golgi apparatus by the transporter associated with antigen processing (TAP) molecule. Once inside the ER or Golgi apparatus, the peptides bind to the MHC class I protein to form a trimolecular complex. This complex is then transported to the cell surface, where it can be recognized by T lymphocyte receptors. Receptors on the surface of a particular type of T lymphocytes, known as CD8⁺ T lymphocytes, specifically recognize the MHC class I complexes that are formed by the combination of MHC class I proteins and peptides derived from a particular protein, and induce the CD8⁺ lymphocytes to kill the cells that bear those complexes. If the protein in question is of viral origin, the T-lymphocytes will thus be specific for cells infected with the relevant virus. In a viral infection many of the MHC molecules of the cell are filled with virus peptides instead of peptides from normal cellular proteins.

The intracellular chain of events leading to formation of MHC class I restricted epitopes for T-cell recognition, e.g. degradation of a native protein, peptide transport into the ER, peptide loading into MHC class I molecules, is referred to as "cellular peptide processing".

The peptides are transported into the ER by an intracellular molecular complex called TAP (2). In the absence of a functional TAP-complex, most MHC class I molecules are retained in the ER, and only a small fraction is transported to the cell surface (3-6). This has been studied in cell lines with defects in the TAP genes. The MHC class I molecules of such cells are often referred to as "empty" or "peptide receptive"; they are unstable at physiological temperature but can be stabilized by culture at low temperature or addition of exogenous MHC class I binding peptides (7-9).

TAP is considered crucial for MHC class I restricted CTL responses, because TAP-deficient cells are inefficiently recognized by conventional MHC class I restricted CTL specific for viral-, minor histocompatibility- or tumour antigens (7, 10, 11). In contrast, TAP-deficient cells can be recognized by some allo-MHC class I specific CTL (10, 12, 13). It is unclear whether such allo-specific CTL recognize MHC class 1 molecules per se, or MHC class 1 molecules loaded with TAP-independent peptides. The latter may include peptide species derived from signal sequences (14, 15), or peptides imported to the ER by other TAP-independent mechanisms.

Tumours are composed of cells, which have lost growth control, i.e. they grow without restraint and can invade normal tissue. Tumours can arise in all types of organs. Many research groups make attempts today to induce T cells to recognize and kill tumour cells. The strategy is to find proteins that are unique for the tumour and peptides from these proteins that can attach to different MHC molecules. Such peptides are then used as components in vaccines that should stimulate the immune response to the tumour. One problem is that different tumours contain different proteins, and that MHC molecules and thereby the peptides that attach to the MHC molecules, vary between individuals, as well as between tumours. Another problem is that many tumours have lost parts of the antigen processing mechanism, e.g. TAP, and, therefore, they are not discovered by conventional T cells. They lack antigenicity, and can escape from the immune response (16-19).

TAP-function is thus considered essential for antigenicity, and it has previously been suggested that inhibition of TAP-function in cells should reduce or abrogate T cell responses to the antigens expressed by the cells. For example, WO 95/15384 describes a TAP inhibitor, a protein ICP47 isolated from Herpes Simplex virus (HSV), and use thereof to inhibit presentation of viral and cellular antigens associated with MHC class I proteins to CD8⁺T lymphocytes. Several investigators emphasize downregulation of TAP and proteasome as a way for cells to escape T-cell mediated recognition. The present invention is based on a novel concept, namely that prevention of cellular peptide processing leads to novel and unique rather than decreased antigenicity of cells due to that the prevention of TAP-function leads to recognition of novel, endogenous MHC class I dependent antigens by host T-cells that are not recognized in the presence of a fully functional TAP-molecule. The inventors have shown that immunization with TAP-deficent cells elicits T-cells directed against epitopes expressed preferentially by TAP-deficient cells and that induction of such T-cells can prevent cancer growth, of several tumour targets.

One application of this invention is immunotherapy of cancer, aimed at eliminating cancer cells with insufficient capacity to process and present TAP dependent peptides. Cancer is a common disease, or rather, group of diseases. Processing/TAP-deficiency has recently been observed in a variety of human tumours (e g cervical carcinoma, melanoma, breast cancer). Furthermore, the development and application of immunotherapy protocols today will be confronted with the problem of escape variants (with e g TAP-defects), which might even be selected by the treatment given. Whether the epitopes turn out to be cell lineage specific or not, the principle may apply to a variety of cancers, since different T-cells against different TAP-deficient tumours may be generated. The principle applied for, may therefore be relevant in immunotherapy treatments given to many hundreds or even thousands of patients per year in Sweden alone.

Another application is the development of a HSV vaccine. HSV is a very common virus, up to a large percentage of the Swedish population is infected, some people have a non-symptomatic but contagious infection. Most persons with symptoms have oral ulcers, which may be very disturbing though they do not affect life-span. An uncommon severe complication is a meningitis which may be life-threatening. HSV can also cause vaginal ulcers which can cause a life-threatening infection in newborn children of infected mothers. It has been shown that HSV downregulate TAP and hence can escape "normal" MHC class I restricted CTL responses . The epitopes and method described may be a way to develop a HSV virus vaccine of which there exist none.

### Summary of the invention

The present invention provides a use of a substance that impairs cellular peptide processing for MHC presentation, the substance being characterized in that tumour cells treated with the substance are subject to specific lysis by CTL elicited by the TAP-deficient variant of said tumour cell which has been transfected with the stimulatory molecule B7-1, in the preparation of a pharmaceutical composition or vaccine for the treatment of cancer or a virus infection, as defined in the claims.

The present invention also provides an in vitro method for providing cells which are capable of activating CD8⁺ T lymphocytes that selectively recognize cells showing impaired cellular peptide processing for MHC presentation comprising the step of treating cells with an effective dose of a substance that impairs cellular peptide processing for MHC presentation, the substance being characterized in that tumour cells treated with the substance are subject to specific lysis by CTL elicited by the TAP-deficient variant of said tumour cell which has been transfected with the stimulatory molecule B7-1, as defined in the claims.

Moreover, the present invention provides an in vitro method for providing immunological effector cells that selectively recognize cells showing impaired cellular peptide processing for MHC presentation comprising the step of stimulating isolated immunological effector cells in vitro with cells provided according to the above-mentioned method.

The following observation shows that antigens or epitopes associated with impaired cellular peptide processing, especially MHC class I dependent, especially antigens associated with impaired TAP-function, can be used for immunization against cancer or as a virus vaccine.

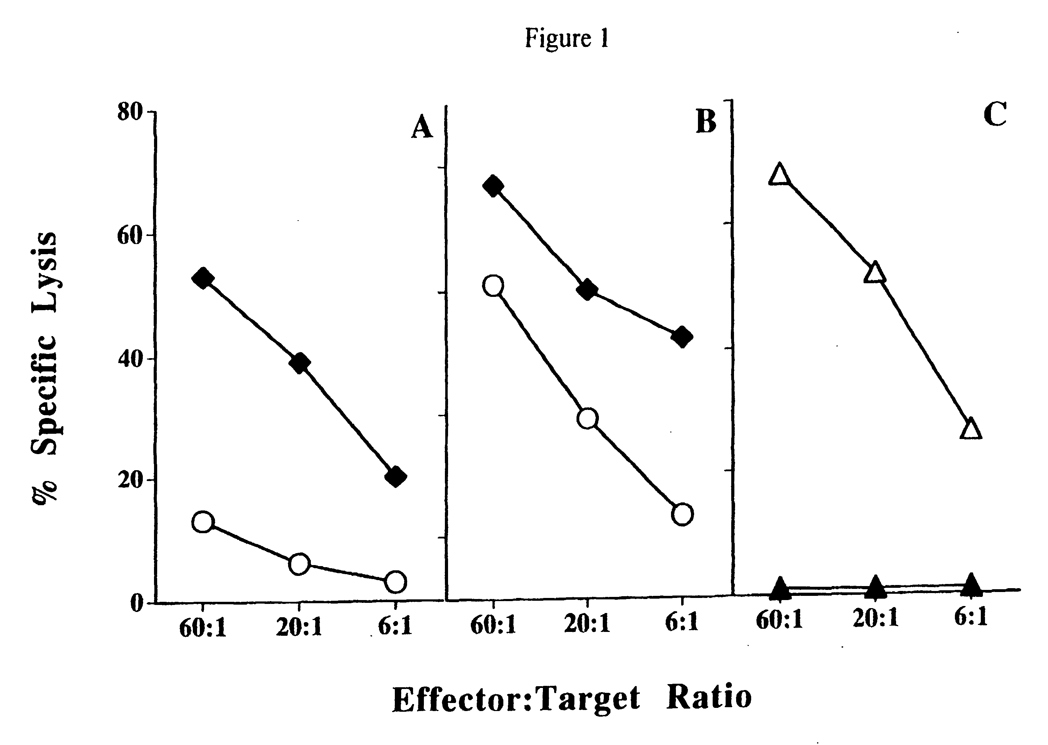

One of the key observations underlying this application is that T-cells could be activated against antigens associated with impaired TAP-function. A TAP-deficient tumour cell from mouse (RMA-S) has previously been produced. This cell line is inefficient in activating responses from cytotoxic T-lymphocytes (example 1, fig 1A and B). After transfection with a stimulatory molecule B7-1 this TAP deficient tumour cell line (RMA-S.B7-1) could activate T cells to a high degree. These T cells recognize a structure independent of TAP, and, therefore, TAP-deficient tumour cells could be killed to a high degree (80% *in vitro).* The inventor has found that TAP-deficient non-transformed normal and tumour cells were capable of inducing a potent CTL response directed against MHC class I dependent epitopes expressed preferentially by TAP-deficient cells. B6 mice immunized with irradiated, B7-1 transfected TAP-deficient tumour cells, were protected from outgrowth of a subsequent transplant of TAP-deficient tumour cells, demonstrating that these novel epitopes associated with impaired TAP-function can serve as tumour rejection antigens *in vivo.*

A human TAP-deficient tumour cell is also killed by T cells elicited by TAP-deficient cells.

Surprisingly, several TAP-expressing murine tumour. cells are also killed by CD8⁺ cells elicited by TAP-deficient cells (5/5 tested, 4 lymphomas and 1 mastocytoma) while non-transformed TAP-expressing cells (proliferating T cells, so called Con A blasts) tested from the same mouse are not killed. It was shown that the CTL recognized epitopes independent of TAP-function on tumours that have TAP-expression, i.e. the tumours can have a relative but not complete impairment of TAP-function.

The inventor demonstrates that the antigens associated with impaired TAP-function can be a shared tumour antigen, which is located on several tumour cell types and hence can be used for tumour immunotherapy.

### Detailed description of the invention

One object of the present invention is the use of substances that impair cellular peptide processing for MHC presentation, such as inhibitors of TAP or of the proteasome, for preparation of a pharmaceutical agent or vaccine that can stop or prevent cancer growth or virus infection by stimulating immunological effectors, especially CD8⁺ cells, preferably cytotoxic cells, directed against antigens or epitopes associated with impaired cellular peptide processing, especially MHC class I dependent, in particular endogenous antigens.

It can be tested that immunological effector cells expand and become activated against cancer or virus by culturing irradiated cells expressing antigens from cancers or viruses alone in the presence of isolated blood fractions, such as a lymphocyte fraction, and testing the effectors for recognition of target cells expressing the antigen, e.g. as is done in (11). That a substance induces expression of antigens or epitopes associated with impaired cellular peptide processing, especially MHC class I dependent, may be determined by using antibodies against MHC class I molecules and measuring MHC class I downregulation, or by recognition by effector cells directed against the antigens. The MHC class I complexes are preferably human complex HLA A, B or C. Antibodies to measure MHC class I expression can e.g. be obtained from PharMingen (San Diego, California). Downregulation of TAP-function can also be measured by peptide transporter assays, e.g. as in (33), and expression of TAP-protein can be measured with antibodies directed against TAP-molecules. Measurement of antibody binding is according to standard techniques e.g. by flow cytometry with a FACS scan analyser (Becton Dickinson).

The substances used according to the invention may be any substance that inhibits the function or the expression of components that take part in the peptide processing of the cell or inhibits an active subfragment of such a substance. More specifically, the invention relates to the use of such substances in eliciting immune responses.

Examples of components that take part in the peptide processing of the cell are e.g. components involved in the translocation of peptides over the ER-membrane such as TAP. Also, substances participating in the cytosolic processing of endogenous proteins such as the proteasome are encompassed.

The substance may be a substance that inhibits the function of TAP such as certain viral proteins e.g. TAP-inhibitors e.g. ICP47 of HSV type 1, IE 12 of HSV type 2. TAP inhibitors may be produced according to WO 95/15384.

The substance may also be one that inhibits the function of proteasome such as proteasome inhibitors such as the peptide aldehyde Z-Leu-Leu-Leu-H (Peptide Internationals Inc., Louisville, KY) or Lactacystin (Calbiochem, La Jolla, California).

Moreover, the substance may be a gene encoding an inhibitor of a substance, that takes part in the peptide processing of the cell e.g. an inhibitor of TAP or proteasome.

The substance may also be one that stops the expression of a substance, that takes part in the peptide processing of the cell e.g. TAP or the proteasome, such as a nucleotide sequence that is complementary at least in part to the RNA or DNA sequences encoding a substance, that takes part in the peptide processing of the cell e.g. antisense oligonucleotides or ribozyme destroying RNA.

Anti-sense polynucleotide sequences or analogues thereof can be used to prevent the expression of proteins in vivo or in vitro. If one adds to a cell a large number of strands of a nucleotide sequence that is complementary to the messenger RNA that is transcribed to produce a particular protein, these "anti-sense" strands will hybridize to the mRNA and limit or prevent its translation. This method could be used to limit or prevent the expression of e.g. TAP and/or proteasome. Also, antisense oligonucleotides that hybridize to DNA could be used (20, 21). Thus it is possible to treat cells with antisense TAP (22). Antisense RNA that hybridizes to mRNA can be provided either by adding RNA to cells or introducing gene sequence transcribing antisense RNA (23). Ribozymes that combine enzymatic processes with the specificity of antisense base pairing may also be used (24). These techniques are discussed in (25).

One purpose provided by the present invention is to stimulate cells in a patient suffering from cancer or certain viruses to express antigens or epitopes associated with impaired cellular peptide processing, especially MHC class I dependent. The virus may be one that impairs peptide processing, e.g. the TAP-function such as Herpes Simplex. This could be done in vitro or in vivo.

When performed in vivo the patient is given substances that impair cellular peptide processing. Thus, compositions containing such a substance e.g. inhibitors of TAP or proteasome may be given. The patient can be vaccinated with e.g. ribozyme, antisense RNA, antisense DNA and/or antisense oligonucleotides against the expression of a substance that takes part in cellular peptide processing or a gene encoding an inhibitor of such a substance i.e. a gene encoding a substance that impairs cellular peptide processing.

DNA can be introduced directly in the cells of a living host by so called DNA immunization. This involves many different techniques e.g. intramuscular injection, intradermal injection (particle bombardment where cells in the epidermis are transfected with DNA-coated gold beads) or delivered by various vectors such as recombinant Shigella. Many other techniques are developed in different laboratories. Also RNA and oligonucleotides may be given in this way (26, 27).

Another object of the invention are cells, that have been treated to express antigens or epitopes associated with impaired cellular peptide processing, especially MHC class I dependent, to be used for preparing a pharmaceutical or vaccine against cancer or virus infections.

These cells may be non-mammalian cells with impaired peptide processing e.g. cells that lack TAP and/or proteasome function and to which human MHC class I molecules have been transfected e.g. insect cells (28).

The invention preferably relates to mammalian cells and especially to autologous mammalian cells that have been treated to express antigens or epitopes associated with impaired cellular peptide processing, especially MHC class I dependent.

The cells may be chosen from hematopoetic cells, especially dendritic cells. Autologous cells are especially preferred.

When cancers are to be treated the cells can also be autologous cells, especially healthy cells from the affected tissue/organ.

These cells, that express MHC class I dependent antigens associated with impaired peptide processing, will then be injected into a patient in order to stimulate T cells to react on these antigens.

Another object of the invention is an in vitro process for induction of antigens or epitopes associated with impaired cellular peptide processing, especially MHC class I dependent, in mammalian cells, characterized in that:
a) the cells are treated with agents that inhibit substances that take place in the cellular peptide processing in mammalian cells e.g. TAP-inhibiting or proteasome inhibiting agents or
b) a sequence that codes for such an inhibiting agent is introduced into the DNA of the cell or
c) a nucleotide sequence that is complementary at least in part to the mRNA or DNA sequences encoding a substance that takes place in the cellular peptide processing in mammalian cells e.g. TAP or proteasome is introduced into the DNA of the cell or
d) the cells are treated with an appropriate ribozyme and
e) when non-mammalian cells are used, transfection thereof with human MHC class I molecules and
f) the cells may be irradiated with an appropriate dose with e.g. γ-irradiation from e.g. Cs¹³⁷.

The steps e) and f) represent parts of standard vaccination techniques, especially with alive cells; they are not part of the invention but may be included in the vaccination process.

In order to make target cells to express a foreign gene product the DNA has to be incorporated in the target cells. Plasmid DNA can be incorporated in the target cells by transfection, e.g. by electroporation, lipofection, calcium precipitation or particle resolution. Vehicles may be needed such as liposomes e.g. DOTAP (Boehringer Manheim).

Another possibility to incorporate in vitro the foreign DNA in the target cells is the use of retroviruses, where the DNA is enveloped in a protein. In the case of retroviruses the DNA will be stably incorporated in the genome with proliferating cells. (29)

Mammalian cells may be cultured in a medium suitable for eucaryotic cells e.g. RPMI 1640 containing bovine serum albumin.

Dendritic cells can be sorted from the peripheral blood by e.g. immunomagnetic sorting to molecules such as CD34 or CD 14. Magnetic beads can be obtained from Dynal. They are grown in vitro in suitable medium, e.g. IMDM (Life Technologies, Inc., Grand Island, NY) with appropriate supplements (30) and various adjuvants to improve development and immunogenicity. Examples of adjuvants are cytokines such as Granulocyte-Macrophage colony stimulating factor (GM-CSF), IL-4, Tumour Necrosis Factor alfa (TNF-alfa), stem cell factor (SCF) or Transforming Growth Factor - beta (TGF-beta), antibodies to MHC Class II or CD40 (which enhance B7 expression) or genes for costimulatory molecules.

Cells, that have been treated to express antigens or epitopes associated with impaired cellular peptide processing, especially MHC class I dependent, e.g. with TAP or proteasome inhibitors, may be used for activation in vivo or in vitro of T cells against MHC class I dependent antigens associated with impaired cellular peptide processing.

Stimulation of T-cells in vitro with dendritic cells is done according to current standard procedures, e.g. T-cells are sorted out from peripheral blood and cultured in the presence of dendritic cells in appropriate media and appropriate additives e.g. MEM media and IL-2 (30, 31).

According to one aspect of the invention lymphoid cells such as T-cells e.g. CTL, preferably CD8⁺ T lymphocytes, activated against antigens or epitopes associated with impaired cellular peptide processing, especially MHC class I dependent, are used for preparing a pharmaceutical or vaccine against cancer or virus infections.

Optimal conditions for human T cells and dendritic cells are e.g. as in (30, 31). T cell activation can be enhanced by treating the cells with cytokines, e.g. GM-CSF or antibodies to e.g. CD40 or MHC class II, which enhance B7 expression. Cytokines and antibodies can be obtained from ImmunoKontakt, Switzerland.

These cells can be tested, e.g. with T-cell clones directed against TAP-inhibited cells. Inhibition of TAP-function can be measured by peptide transporter assays, e.g. as in (32-34).

The invention also relates to a kit, for use in a process for induction of antigens or epitopes associated with impaired cellular peptide processing, especially MHC class I dependent, in cells, characterized in that it comprises an active dose of a substance that stimulates formation of antigens or epitopes associated with impaired cellular peptide processing, especially MHC class I dependent, such as an inhibitor of TAP or of proteasome or a nucleotide sequence that is at least in part complementary to the mRNA or DNA sequences encoding proteasome or TAP as a first component, and cytokines, genes for cytokines, costimulatory molecules, gold beads and/or liposomes as a second component.

The invention also concerns a pharmaceutical composition or a vaccine comprising a pharmaceutically effective dose of a substance that induces antigens or epitopes associated with impaired cellular peptide processing, especially MHC class I dependent, such as an inhibitor of TAP or of proteasome or a gene encoding a proteasome or a TAP inhibitor or a nucleotide sequence that is complementary at least in part to the mRNA or DNA sequences encoding proteasome or TAP e.g. antisense nucleotides or ribozyme together with a pharmaceutically acceptable adjuvant. selected from cytokines, genes for cytokines, costimulatory molecules, gold beads and/or liposomes and optionally pharmaceutical acceptable additives.

Without being subject of the claims, the present invention provides the following methods for treatment, prevention and diagnosis of cancer and virus infections:
a) cells taken out of the body of a human being are treated with inhibitors of cellular peptide processing, e.g. TAP-inhibitors, and are readministered to the body possibly together with a pharmaceutically acceptable adjuvant, or
b) cells that express antigens or epitopes associated with impaired cellular peptide processing, especially MHC class I dependent, are administered to the body possibly together with a pharmaceutically acceptable adjuvant, or
c) autologous T-cells are stimulated in vitro against antigens or epitopes associated with impaired cellular peptide processing, especially MHC class I dependent, and are administered to the body, or
d) inhibitors of cellular peptide processing for MHC class I presentation are administered to the body, such as TAP-inhibitors, together with a pharmaceutically acceptable adjuvant.

The cells are preferably autologous mammalian cells such as those mentioned herein, e.g. on page 10, line 5 to page 10, line 17.

The T-cells may be stimulated with cells that have been treated to express antigens or epitopes associated with impaired cellular peptide processing, especially MHC class I dependent, such as the cells mentioned herein, e.g. on page 9, line 25 to page 10, line 17. They may be autologous.

The amount of target cells, stimulated T-cells or inhibitors is a pharmaceutically effective amount that can be determined by the practitioner or doctor.

Adjuvants are substances that increase the effect of pharmaceutical substances, such as those mentioned on page 11, line 27-31.

Pharmaceutical compositions of the present invention contain a physiologically acceptable carrier together with at least one substance that impairs cellular peptide processing as described herein, dissolved or dispersed therein as an active ingredient.

As used herein, the term "pharmaceutically acceptable" represents that the materials are capable of administration to or upon a human without the production of undesirable physiological effects such as nausea, dizziness, gastric upset and the like.

The preparation of a pharmacological composition that contains active ingredients dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol. ethanol or the like and combinations thereof. In addition, if desired, the composition can contain minor amounts of auxiliary substances such as wetting or emulsifying agents, pH buffering agents and the like which enhance the effectiveness of the active ingredient.

The pharmaceutical composition of the present invention can include pharmaceutically acceptable salts of the components therein. Pharmaceutically acceptable salts include the acid addition salts that are formed with inorganic acids such as, for example, hydrochloric or phosphoric acids, or such organic acids as acetic, tartaric, mandelic; inorganic bases such as, for example, sodium, potassium, ammonium, calcium or ferric hydroxides, and such organic bases as isopropylamine, trimethylamine, 2-ethylaminoethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water. Exemplary of such additional liquid phases are glycerine, vegetable oils such as cottonseed oil, organic esters such as ethyl oleate, and water-oil emulsions.

The following observations show substances that impair cellular peptide processing, especially MHC class I dependent, can be used as agents for immunization against cancer.

Summary of the observations:
- CD8⁺ T-cells can be activated against epitopes recognized preferentially expressed by TAP-deficient cells. This is shown by immunizing B6 mice with syngenic TAP-deficient tumour cells transfected with a T cell activatory molecule (TAP-deficient RMA-S tumour cells transfected with the costimulatory molecule B7- 1. The cells are designated RMA-S.B7-1) and non-transformed spleen cells and dendritic cells from TAP1 -/- mice (both alleles of the TAP gene knocked out) and testing the elicited CTL on different TAP-deficient and TAP-expressing target cells (Examples 1-3).
- The recognition of epitopes is dependent of MHC class I expression. This is shown by testing elicited CTL on cells expressing different combinations of MHC class I defects (Example 2).
- The epitopes are recognized on TAP-deficient murine tumour cells and murine TAP-deficient non-transformed cells as well as on a TAP-deficient human tumour cell line (Examples 2-3).
- Surprisingly, syngenic TAP-deficient cells, both RMA-S.B7-1 tumour cells as well as spleen cells and dendritic cells from TAP1 -/- mice, elicit T cells that recognize several different syngenic TAP-expressing murine tumour cells while syngenic TAP-expressing non-transformed cells are not killed (Examples 6-8).
- The epitopes recognized on TAP-expressing tumour cells are epitopes independent of TAP-function (Example 6).
- Immunization with syngenic TAP-deficient cells can protect against tumour growth in vivo of both TAP-deficient and TAP-expressing tumour cells (Examples 5, 9).

In conclusion, the inventor demonstrates that the antigens associated with impaired TAP-function can be a shared tumour antigen, which is located on several tumour cell types and hence can be used for tumour immunotherapy.

In the experiments B6 mice are immunized with syngenic TAP-deficient cells e.g. cells from TAP1-/- mice where the TAP1 gene has been deleted by genetic manipulation. In a living human being this is not possible yet. However, cells treated with antisense oligonucleotides against TAP have the same cellular phenotype as cells where TAP-function has been abrogated by structural genetic changes as in the mutant cell line RMA-S or cells from mice where TAP has been deleted by genetic manipulation (TAP knock-out mice) (22). This phenotype is characterized e.g. by decreased cell surface expression of MHC class I molecules, which can be upregulated by incubation at 26°C, also by an enhanced stimulatory capacity to externally added antigens. The key difference between the invention and such studies is that the inventors have found that TAP-inhibition adds novel endogenous MHC class I dependent antigens for CD8⁺ T cell recognition. It is further shown that these are recognized on a variety of different tumour cells. Since the human cell line T2K^{b} is recognized by the elicited CTL, the epitopes associated with impaired TAP-function can also be recognized on human cells. In the human, autologous or MHC matched cells can be treated with TAP-inhibitors of different kinds; these cells can be used to elicit CTL against antigens or epitopes associated with impaired cellular peptide processing, especially MHC class I dependent. According to the data presented these CTL may be used in immunotherapy of both TAP-deficient and TAP-expressing tumours. From the literature it is also known that several viruses can inhibit antigen presentation and hence escape conventional CTL recognition. For example, Herpes Simplex inhibits TAP-function (33, 34). Therefore CTL elicited by TAP-inhibited cells may also be used as a therapeutical agent in viral infections where TAP-function is inhibited as above. The presentation of MHC class I molecules and TAP-dependent peptides at the cell surface is a complex process depending on many factors, of which translocation of peptides over the ER-membrane by TAP-molecules is one. Another crucial step is cytosolic processing of endogenous proteins by the proteasome. Cells that lack components of the proteasome have a similar phenotype to TAP-deficient cells, i.e. deficient generation of antigenic peptide. Proteasome inhibitors block MHC class I restricted antigen presentation and assembly of MHC class I molecules, the assembly can be reconstituted by exogenously added peptides (1, 35-37). It is likely that the epitopes associated with impaired TAP-function will be exposed also upon inhibition of the proteasome and could with a more general term be named MHC class I dependent epitopes associated with impaired cellular peptide processing. Therefore the invention also relates to the elicitation of T cells with proteasome inhibitors. It is also easy to envisage that other yet undefined factors are necessary for formation of the MHC/TAP-dependent-peptide complex and that inhibition of those factors will result in a similar phenotype exposing epitopes associated with impaired TAP-function.

This underlying discovery reveals the existence of MHC class I dependent CTL epitopes that are recognized preferentially in the absence of a functional TAP complex. B6 mice immunized with B7-1 transfected TAP-deficient RMA-S tumour cells or spleen cells from TAP1 -/- mice, generated a potent CTL response against both RMA-S tumour cells and TAP1 -/- Con A blasts targets. In contrast, TAP-expressing RMA-S.TAP2 tumour cells and B6 Con A blasts were largely resistant to lysis by these CTL. RMA-S.B7-1 immunized mice were protected from outgrowth of RMA-S tumour transplants, indicating that these epitopes associated with impaired TAP-function can serve as tumour rejection antigens in vivo. Surprisingly, several TAP-expressing tumours were also sensitive to the elicited CTL while non-transformed cells were resistant It was shown that the CTL recognized epitopes independent of TAP-function on tumours that still have TAP-expression, i.e. they can have a relative but not complete impairment of TAP-function. The difference in sensitivity between RMA and RMA-S.TAP2 which are both TAP-expressing is probably due to that RMA-S.TAP2 is a transfectant with TAP and hence can overexpress TAP compared to the levels in the original tumour RMA.

In the experiments described B6 mice have been immunized with syngenic TAP-deficient cells. A human correlate is to elicit CTL with TAP-deficient cells, e.g. autologous cells to which TAP-inhibitors have been introduced. According to the data presented these CTL may be used in immunotherapy of both TAP-deficient and TAP-expressing tumours. These CTL may also be used as therapeutical agent in viral infections where TAP-function is inhibited (33, 34). This study is done with several different tumour cells of lymphoid origin. Also a non-lymphoid cell line, the H-2Kb transfected mastocytoma P815 is killed by the elicited CTL.

The agents according to the invention may be used against tumours, preferentially that have lost expression of TAP, but also towards TAP-expressing tumours. The agents may also be used against certain virus infections where TAP-function is inhibited by viral proteins, e.g. in Herpes Simplex virus infected cells.

### Brief description of the drawings:

### Figure legends

**Figure 1.** Immunization of 136 mice with RMA-S.B7-1 cells elicits CD8⁺ CTL that recognize non-transfected RMA-S cells. (A and B) B6 mice were immunized in vivo and splenocytes were restimulated in vitro with RMA-S tumour cells (O) or RMA-S cells transfected with B7-1 (◆) and tested for cytotoxicity against RMA-S target cells. Two experiments are shown, panel A is representative of 4/6 experiments, panel B is representative of the remaining experiments. (C) CTL generated as above were depleted with anti-CD8 antibodies and complement (▲) or anti-CD4 antibodies and complement (Δ) and tested for cytotoxicity against RMA-S target cells. One representative experiment out of three is shown.

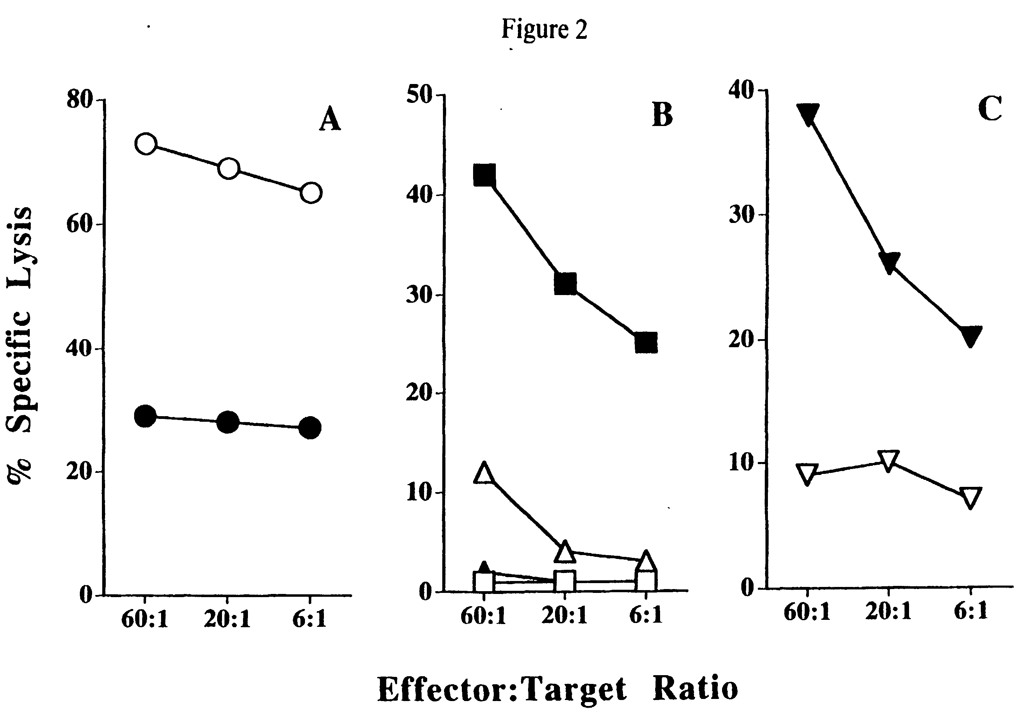

**Figure 2.** Recognition of epitopes by RMA-S.B7-1 elicited CTL requires the absence of TAP-function and the presence of MHC class I molecules in the target cell. B6 mice were immunized in vivo and splenocytes were restimulated in vitro with RMA-S.B7-1 cells and tested for cytotoxicity against (A) RMA-S (○) and RMA-S.TAP-2 (●), (B) Con A blasts from B6 (□), TAP1 -/- (■), β₂m -/- (Δ), and TAP1/β₂m -/- mice (▲) and (C) T2 (∇) and T2K^{b} (▼) cells. One representative experiment out of three is shown.

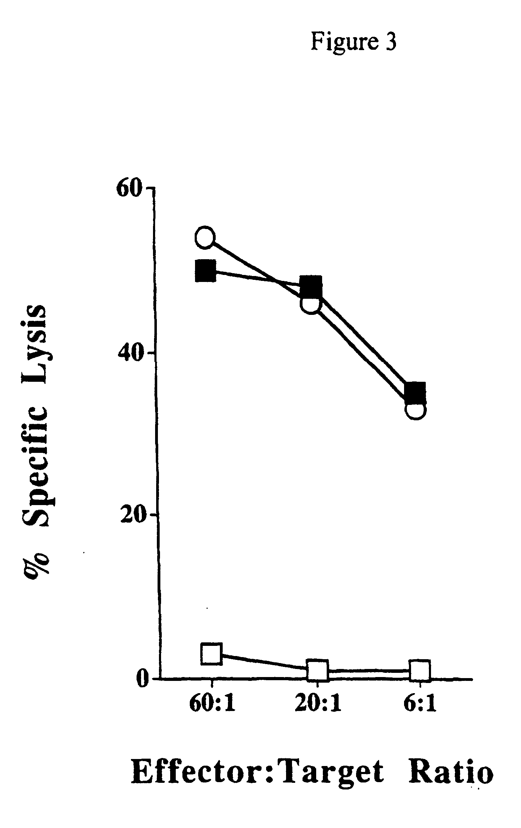

**Figure 3.** Immunization with TAP1 -/- splenocytes elicits CTL that recognize TAP1 -/- Con A blasts and RMA-S tumour cells. B6 mice were immunized in vivo and splenocytes were restimulated in vitro with splenocytes from TAP1 -/- mice and tested for cytotoxicity against RMA-S (○), B6 Con A blasts (□) and TAP1 -/- Con A blasts (■). One representative experiment out of four is shown.

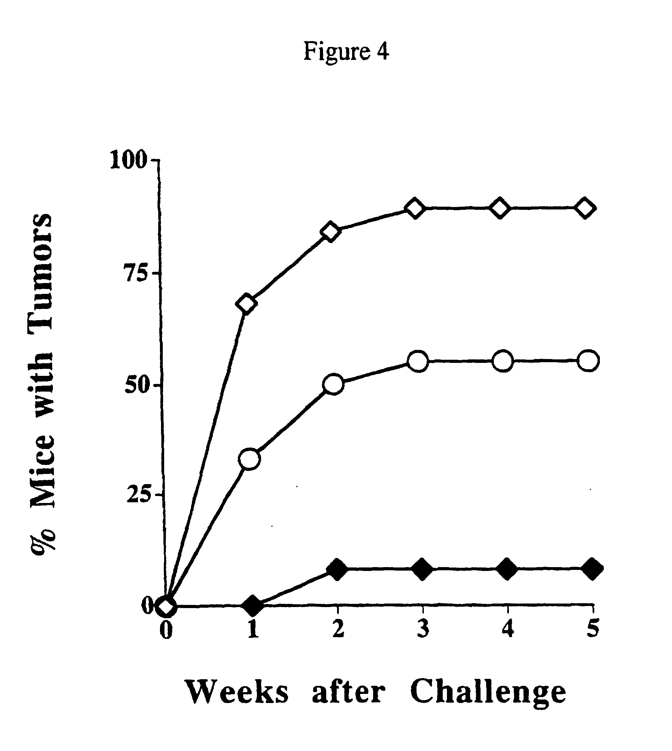

**Figure 4.** Immunization of B6 mice with RMA-S.B7-1 cells protects from outgrowth of RMA-S tumour cells. B6 mice were given 10⁶ live RMA-S tumour cells after immunization with PBS ( ), RMA-S (○) or RMA-S.B7-1 (◆). The figure represents the accumulated data of four separate experiments (three for RMA-S.B7-1) with 4-6 mice/immunization group in each experiment.

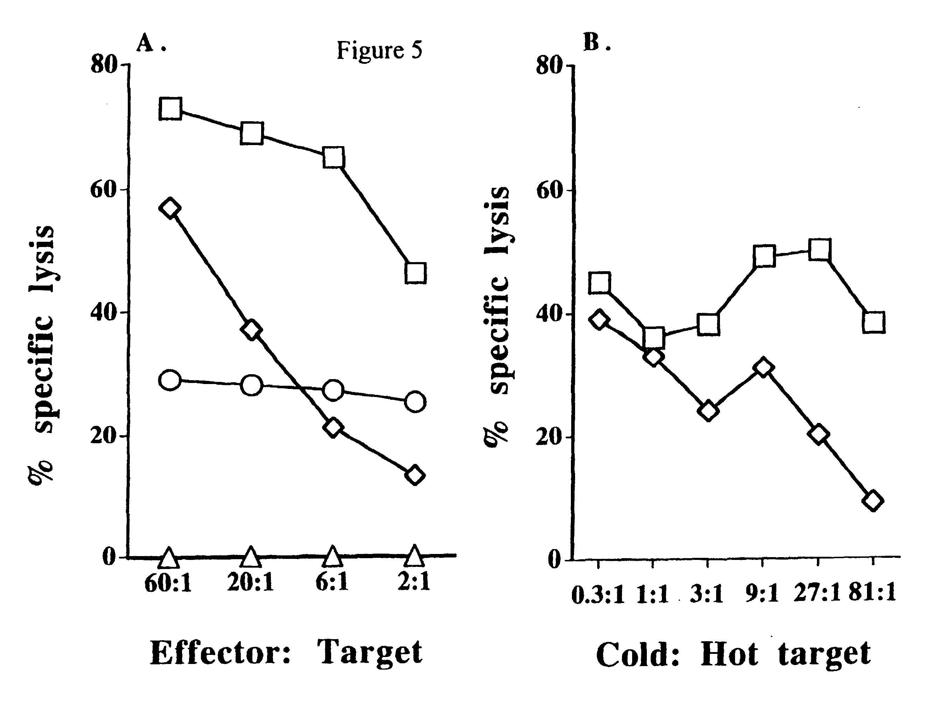

**Figure 5.** The TAP-expressing tumour RMA expresses epitopes independent of TAP-function. B6 mice were immunized in vivo and splenocytes were restimulated in vitro with RMA-S.B7-1 tumour cells and tested for cytotoxicity against (A) RMA-S (□), RMA ( ), RMA-S.TAP2 (○) or B6 Con A blasts (Δ), (B) RMA to which cold B6 Con A blasts (□) or TAP1-/- Con A blasts( ) in designated ratios were added.

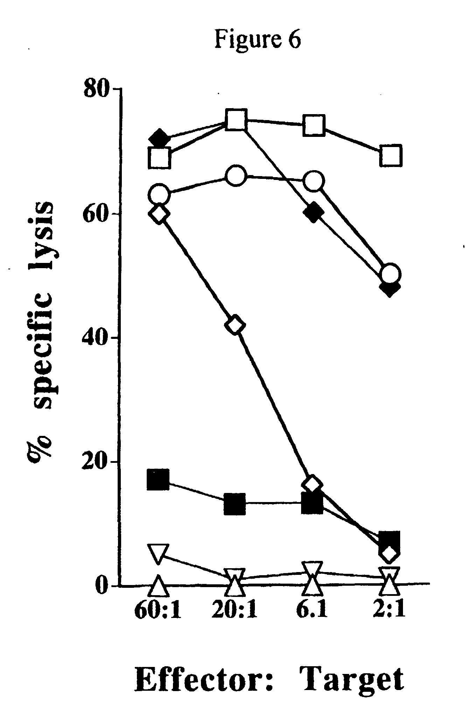

**Figure 6.** Several TAP-expressing tumour cell lines are killed by CTL elicited by RMA-S.B7-1, while non-transformed TAP-expressing cells are resistant. B6 mice were immunized in vivo and splenocytes were restimulated in vitro with RMA-S.B7-1 tumour cells and tested for cytotoxicity against RMA-S (□), EL-4 ( ), ALC (○), C4425- (Δ), P815 (∇), B6 Con A blasts (■) and TAP1-/- Con A blasts (◆). One representative experiment out of three is shown.

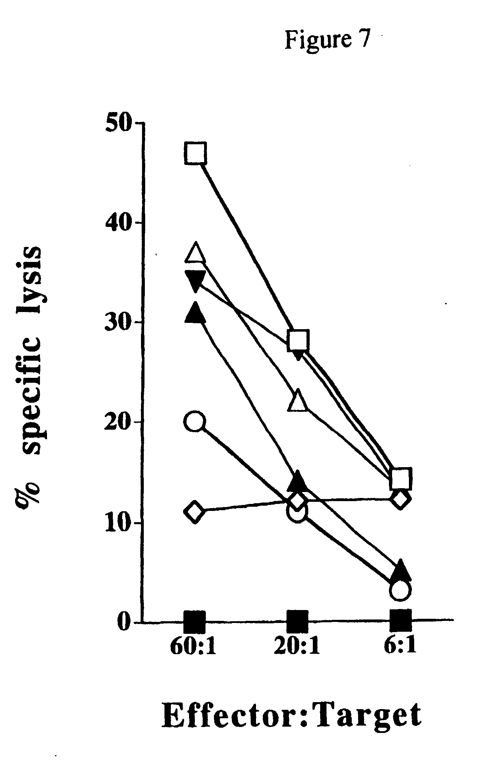

**Figure 7.** Immunization with TAP1 -/- dendritic cells elicits CTL that recognize several TAP-expressing tumour cells. B6 mice were immunized in vivo with dendritic cells from TAP 1 -/- mice and splenocytes were restimulated in vitro with splenocytes from TAP 1 -/- mice and tested for cytotoxicity against RMA-S (□), EL-4 ( ), ALC (○), RMA (Δ), P815K^{b} (▲), 26E1Nmyc (▼) and B6 Con A blasts (■). One experiment of two is shown.

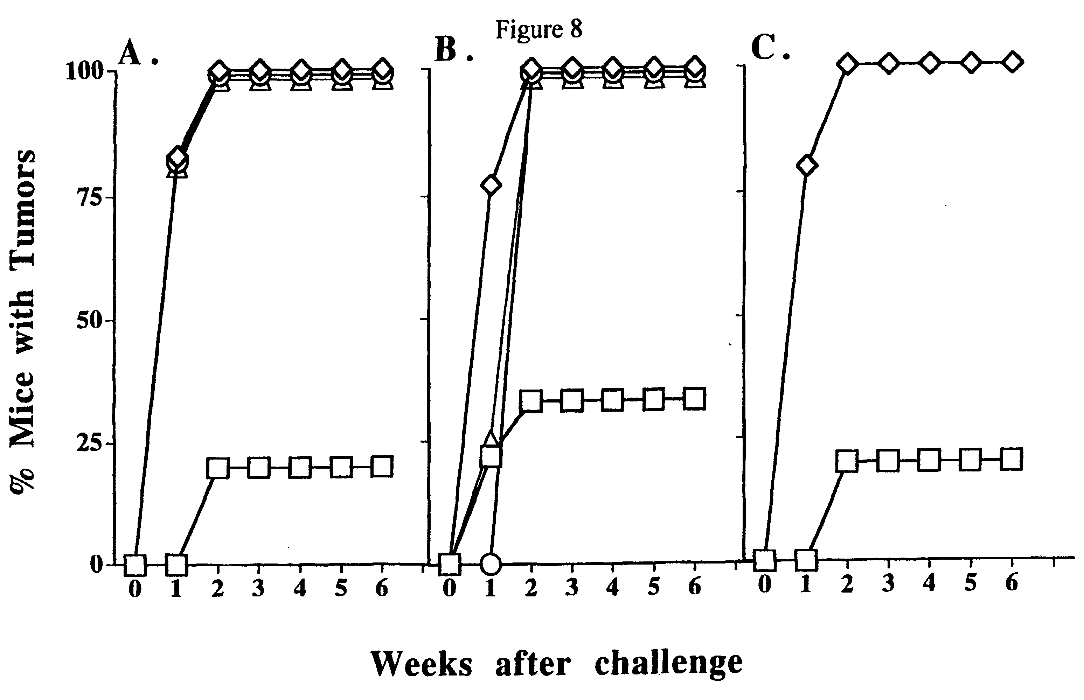

**Figure 8.** Immunization of 136 mice with RMA-S.B7-1 cells protects from outgrowth of several TAP-expressing tumour cells. B6 mice were immunized with PBS ( ) or RMA-S.B7-1 (□), or CD8-/- mice were immunized with PBS (Δ) or RMA-S.B7-1 cells (○) and given in (A) 10⁵ RMA tumour cells, (B) 10² EL-4 tumour cells (C) 10³ ALC tumour cells. The figure represents one experiments with 4-6 mice/immunization group.

The invention will now be described by reference to some non limiting examples

All technical and scientific terms used herein are, unless otherwise defined, intended to have the same meaning as commonly understood by one of ordinary skill in the art. Techniques employed herein are those that are known to one of ordinary skill in the art unless stated otherwise.

### Materials and Methods

*Mice.* All mice were bred and maintained at the Microbiology and Tumour Biology Center, Karolinska Institute. The generation and characterization of TAP1 -/-, β₂-microglobulin (β₂m) -/- and TAP1/β₂m -/- mice has been described (38-40). The TAP 1 -/- and β₂m -/- mice used in the present study have been backcrossed to B6 (C57BL/6) at least six times. Animal care was in accordance with institutional guidelines.

*Cell lines.* T2K^{b} is a H-2K^{b} (mouse MHC allele (type)) transfected subline of the TAP1/2 deficient mutant human cell line T2 (41). All cell lines were grown in RPMI 1640 medium (Life Technologies, Gaithersburg, MD) supplemented with penicillin-streptomycin and 5% FCS (Fetal Calf serum) at 37°C and 5% CO₂.

*Antibodies.* B7-1 (RMA-S.B7-1) expression was assessed either with the CTLA-4Ig fusion protein (42), a kind gift from Dr P. Lane, Basel Institute for Immunology, Basel, Switzerland, and a FITC (Fluoroscein isothiocyanate)-conjugated anti-human IgG antibody (Dako, Glostrup, Denmark), or with a biotinylated anti-B7-1 monoclonal antibody 16-10A1 (PharMingen, San Diego, CA) and NEUTRALITE avidin-FITC (Southern Biotechnology Associates Inc., Birmingham, AL).

*Cold target competition assay.* For the cold target competition assay cold (unlabeled) Con A blasts were incubated at different concentrations with a constant number of effector cells and 5 x 10^{3 51}Cr-labeled target cells.

*Immunization with bone marrow derived dendritic cells.* Bone marrow derived derived dendritic cells were obtained from TAP1-/- mice using the protocol of Inaba and colleagues (43) with the following alterations. Bone marrow cells were cultured in Dulbecco's modified Eagles Medium containing 10 % supernatant from the GM-CSF (Granulocyte Macrophage Colony Stimulating Factor secreting cell line X63 (a kind gift from Dr C.Watts, Univ Dundee, Dundee, UK) and 20 % FCS. The culture media was replaced every third day, and the cells were replated on day 7. On the eight day, the non-adherent cells were used for in vivo immunization. 10⁵ cells were given intraperitoneally, splenocytes were restimulated 10 days after in vivo immunization.

### Examples

A. CD8⁺ T cells can be elicited to MHC class I dependent epitopes associated with impaired TAP-function. Immunization with a TAP-deficient cell can protect against tumour growth in vivo.

### Example 1

### RMA-S cells transfected with B7-1 (CD80) elicit CTL that recognize non-transfected RMA-S cells.

B6 mice were immunized with three weekly s.c. injections of 10⁷ irradiated (100 Gray (Gy)) tumour cells. Tumours used were serially passaged as ascites cell lines in 4 Gy irradiated mice. The tumour cells were TAP-2 deficient ones, called RMA-S cells (derived from the Rauscher leukaemia virus-induced mouse T-cell lymphoma RBL-5 of B6 origin (44)), which were transfected with B7-1, i.e. 2 x 10⁶ RMA-S cells were incubated with 10 µl LIPOFECTAMINE (Life Technologies, Gaithersburg, MD) and 1 µg of the murine B7-1 gene cloned in a pSRIneo plasmid (45), a gift from Bristol Meyers Inc, Seattle, to Prof Klas Kärre. Transfected cells were selected on GENETICIN (Life Technologies, Gaithersburg, MD) at a concentration of 1 mg/ml. The 1 % most positive fraction of the B7-1 expressing RMA-S cells were sorted on a FACS VANTAGE cell sorter (Becton Dickinson, Mountain View, CA) and designated RMA-S.B7-1.

This transfection resulted in strong and reproducible lysis of non-transfected RMA-S targets (Fig. 1 A and B). The lysis could be abrogated by pre-treatment of effectors with anti-CD8 antibodies (CD8 is a marker of the cell surface of CTL) and complement (Fig. 1 C). The complement-mediated depletion of effector populations was conducted as follows: 20 x 10⁶ effector cells were incubated in 200 µg of anti-CD8 antibody (a mixture of 169.4 and 156.7.7 in 1 ml PBS, or 200 µg of anti-CD4 antibody (YTS191) in 1 ml PBS for 60 min at 4°C. All antibodies were generously provided by Dr H. Waldman, University of Cambridge, Cambridge, UK. The cells were washed once and incubated with rabbit complement (Pel-Freeze Biologicals, Brown Deer, WI) diluted 1:8, for 75 min in 37°C.

This demonstrates that it is possible to generate CTL against syngenic TAP-deficient cells. Control cell lines, YAC-1 (a Moloney virus induced T cell lymphoma of A/Sn background) and P815 (a Methylcholantrene induced mastocytoma of DBA/2 background), as well as Concanavalin A (Con A) blasts from BALB/c mice were resistant to lysis by these CTL (data not shown). The generation of Con A-activated T-cell blasts was as follows: Spleen cells were incubated for 48 h at 2 x 10⁶ cells/ml in α-MEM medium (Life Technologies, Gaithersburg, MD) supplemented with penicillin-streptomycin, 10 % FCS, 10 mM HEPES (Life Technologies, Gaithersburg, MD), 3 x 10⁻⁵ M 2-ME (2-Mercaptoethanol) (Sigma, St. Louis, MO) and 3 µg/ml of Con A (Sigma, St. Louis, MO). Before use as targets in a standard 4 h ⁵¹Cr cytotoxicity assay, dead cells were removed by centrifugation on a LYMPHOPREP gradient (Nycomed, Oslo, Norway).
TAP-2 deficient RMA-S tumour cells were inefficient in eliciting cytotoxic responses in B6 mice (Fig. 1 A and B).

### Example 2

### CTL recognition requires the presence of MHC class I molecules and the absence of TAP in the target cell.

A TAP-2 transfectant of RMA-S (RMA-S.TAP2 cells, also referred to as RMA-S II 5.9 cells, were derived by transfection of RMA-S with the murine TAP-2 gene (46)) was virtually resistant to lysis by RMA-S.B7-1 elicited CTL (Fig. 2 A) as in Example 1. This indicated that the epitopes were recognized preferentially in cells devoid of TAP expression. In line with this, Con A blasts from TAP1 -/- mice were highly sensitive to lysis, whereas Con A blasts from B6 mice were resistant to lysis. Con A blasts from TAP1/β₂m -/- (double mutant) mice were resistant to lysis, suggesting an MHC class I dependence in the CTL recognition of epitopes (Fig. 2 B).

Indeed, the human TAP-deficient cell line T2 transfected with H-2K^{b} was sensitive to lysis by RMA-S.B7-1 elicited CTL, while non-transfected T2 cells were resistant (Fig. 2 C). Taken together, these results indicate that at least part of the response elicited by RMA-S.B7-1 is MHC class I specific or restricted and directed against epitopes expressed preferentially, if not exclusively, by TAP-deficient cells. At least some of the epitopes could be recognized on both non-transformed and transformed lymphoid cells, and on cells of both human and murine origin. These epitopes will be referred to as epitopes associated with impaired TAP-function.

### Example 3

### TAP1 -/- splenocytes elicit CTL that recognize TAP1 -/- Con A blasts and RMA-S tumour cells.

As shown above, CTL elicited by RMA-S.B7- 1 killed TAP1 -/- Con A blasts. Accordingly, immunization of B6 mice with splenocytes from TAP1 -/- mice yielded cytotoxic cells that efficiently lysed TAP1 -/- Con A blasts and RMA-S tumour cells while B6 Con A blasts and RMA-S.TAP2 were killed at markedly reduced levels (Fig. 3; data not shown). The killing of the TAP-deficient cells was also seen with effectors from mice depleted of NK (natural killer) cells (data not shown). B6 mice were immunized with two weekly s.c. injections of 50x10⁶ irradiated (20 Gy) spleen cells.

### Example 4

### Generation of primary CTL by the epitopes associated with impaired TAP-function.

Stimulation in vitro, without prior in vivo immunization, of B6 spleen cells with RMA-S.B7-1 and TAP1 -/- spleen cells was conducted as follows: Single cell suspensions of spleens from immunized or non-immunized mice were prepared. 20 x 10⁶ effector cells were incubated with 2 x 10⁶ irradiated tumour cells or 20 x 10⁶ irradiated spleen cells. Cultures were kept in 10 ml of RPMI 1640 medium supplemented with penicillin-streptomycin, 10 % FCS, 3 x 10⁻⁵ M 2-ME, 1 mM sodium pyruvate, 0,1 mM non-essential amino acids and 2 mM L-glutamine at 37° C and 5 % CO₂ for five days.

This stimulation reproducibly resulted in cytotoxic responses against RMA-S and TAP1 -/- Con A blasts targets, while RMA-S.TAP2 and B6 Con A blasts were considerably less sensitive. The in vitro cytotoxicity assay was conducted as follows: Target cells were labelled with ⁵¹Cr and resuspended in cell culture medium. 5 x 10³ target cells were added to each well followed by addition of effector cells. The cells were incubated for 4 h at 37°C and supernatants were harvested. Radioactivity was measured in a Pharmacia-LKB γ-counter, and specific lysis was calculated [(CPM (counts per minute) released with effector cells-CPM released without effector cells) / (CPM released by detergent-CPM released without effector cells)] x 100. Results with more than 20 % spontaneous lysis were discarded.

Stimulation in vitro with non-transfected RMA-S cells yielded in some experiments lower levels of lysis (Table I; data not shown). The killing of RMA-S and TAP1 -/-Con A blast targets was abrogated by pre-treatment of effectors with anti-CD8 antibody and complement (data not shown).

### Example 5

### Immunization of B6 mice with RMA-S.B7-1 protects against RMA-S tumour growth.

To address whether it was possible to elicit a protective immune response against a TAP-deficient tumour cell line, we immunized B6 mice with RMA-S cells, RMA-S.B7-1 cells or PBS (phosphate buffered saline). After three weekly immunizations, mice were challenged with 10⁶ live RMA-S tumour cells s.c., a dose previously found to overcome the NK mediated rejection of RMA-S (41). The in vivo tumour growth was as follows: B6 mice were immunized as described. One week after the last immunization, mice were given 10⁶ live tumour cells s.c. and growth was followed weekly by palpation. For each mouse, the experiment was terminated when the tumour reached a diameter of 20 mm.

Eighty nine percent of the mice (17/19 mice) immunized with PBS developed progressively growing tumours within three weeks after challenge. In contrast, only eight percent (1/13) of the mice immunized with RMA-S.B7-1 developed tumours. Mice immunized with RMA-S were partially protected: Fifty five percent of the mice (10/18 mice) developed progressively growing tumours (Fig. 4).

The RMA-S.B7-1 mediated protection from tumour growth was also seen in NK 1.1. depleted mice (data not shown).

B. Epitopes associated with impaired TAP-function is a shared tumour antigen found on several TAP-expressing tumours but not on non-transformed cells. Immunization with a TAP-deficient cell protects in vivo against tumour growth of TAP-expressing tumour cells.

### Example 6

### The TAP-expressing tumour cell RMA is recognized by CTL directed to epitopes associated with impaired TAP-function.

Surprisingly, CTL elicited by RMA-S.B7-1 reproducibly killed the TAP-expressing parental tumour cell of RMA-S, RMA. The lysis levels were markedly higher than with the TAP-transfected tumour cell line RMA-S.TAP2, but still below the levels of lysis of the TAP-deficient cell RMA-S (Fig 5A). As described above, no killing was observed of TAP-expressing B6 Con A blasts. In cold target inhibition experiments with Con A blasts from B6 and TAP-/- mice, the latter were more efficient in inhibiting lysis of RMA by CTL elicited by RMA-S.B7-1 (Fig 5B), demonstrating that at least part of the killing of RMA by these CTL was dependent on recognition of epitopes independent of TAP-function. Control experiments where hot and cold targets were incubated without effectors did not result in any lysis by either cold target (data not shown).

### Example 7

### Several TAP-expressing tumour cell lines are killed by CTL elicited by RMA-S.B7-1 while non-transformed TAP-expressing cells are resistant.

A possible explanation to the observed killing of RMA is that some tumour cells may have a relative deficiency in TAP-function, i.e. they express suboptimal levels of TAP-proteins. To investigate whether epitopes associated with impaired TAP-function could be a shared antigen we tested other H-2^{b} expressing tumour cell lines transformed by different agents, for killing by RMA-S.B7-1 elicited CTL. Indeed, all tumour cells tested, the carcinogen induced (9,10-dimethyl-1,2-benzanthracene) EL-4 (American Type Culture Condition, Rockville, MD), the Radiation Leukaemia virus induced ALC (generously provided by Dr Wen Tao, Karolinska Institute, Sweden) and 26E-1Nmyc tumour cells (a gift from Dr Santiago Silva, Karolinska Institute, Sweden) were killed by RMA-S.B7-1 elicited CTL. 26E1Nmyc is a spontaneous lymphoma from mice transgenic for EBNA-1 and N-myc (derived by crossing EBNA-1 and N-myc transgenic mice (47, 48)). A β₂m-negative variant of EL-4, as well as P815 tumour cells (a mastocytoma of a H-2^{d} origine obtained from American Type Culture Condition, Rockville, MD) were not killed by RMA-S.B7-1 elicited CTL (Fig 6). Of the cells described above, only H-2^{d} positive P815 and 26E-1Nmyc cells were killed by B6 CTL elicited by Balb/c splenocytes (data not shown) showing that the tumour cells were not generally sensitive for all CTL tested.

### Example 8

### TAP-deficient nontransformed cells elicit CTL that recognize several TAP-expressing tumour cell lines but not TAP-expressing non-transformed cells.

CTL from B6 mice immunized with splenocytes or cultured dendritic cells from TAP1 -/- mice also killed RMA, ALC, 26E-1Nmyc, EL-4 (to lower levels), P815 cells transfected with H-2K^{b} and TAP1 -/- Con A blasts. B6 Con A blasts were not killed by these CTL (Fig 7). This strengthens the notion that TAP-expressing tumour cells but not non-transformed cells express epitopes associated with impaired TAP-function. The lysis levels were lower than those observed with RMA-S.B7-1 which is probably due to the high levels of 137 expression on RMA-S.B7-1.

### Example 9

### Immunization with RMA-S.B7-1 protects from tumour growth of several TAP-expressing tumours.

B6 mice were immunized with RMA-S.B7-1 or non-immunized. One week after the last immunization mice were given 10⁵ RMA tumour cells or 10² EL-4 tumour cells or 10³ ALC tumour cells. In the immunized groups only 20 % of mice developed tumours while all mice in the non-immunized groups developed progressively growing tumours. For RMA and EL-4 the protection observed in the immunized mice was not seen in mice deficient of CD8, demonstrating that this protection was mediated by CD8⁺ CTL (Fig 8 A-C).

### References

1. Groettrup, M., A. Soza, U. Kuckelkorn and P.-M. Kloetzel. 1996. Peptide antigen production by the proteasome: complexity provides efficiency. *Immunol. Today.* 9:429-435.
2. Heemels, M.T, and H. Ploegh. 1995. Generation, translocation, and presentation of MHC class I-restricted peptides. *Annu. Rev. Biochem.* 64:463-491.
3. Powis, S.J., A.R. Townsend, E.V. Deverson, J. Bastin, G.W. Butcher, and J.C. Howard. 1991. Restoration of antigen presentation to the mutant cell line RMA-S by an MHC-linked transporter. *Nature.* 354:528-531.
4. Spies, T, and R. DeMars. 1991. Restored expression of major histocompatibility class I molecules by gene transfer of a putative peptide transporter. *Nature.* 351:323-324.
5. Degen, E., M.F. Cohen-Doyle, and D.B. Williams. 1992. Efficient dissociation of the p88 chaperone from major histocompatibility complex class I molecules requires both β₂-microglobulin and peptide. *J*. *Exp. Med.* 175:1653-1661.
6. Suh, W.K., E.K. Mitchell, Y. Yang, P.A. Peterson, G.L. Waneck, and D.B. Williams. 1996. MHC class I molecules form ternary complexes with calnexin and tap and undergo peptide-regulated interaction with TAP via their extracellular domains. *J. Exp. Med*. 184:337-348.
7. Townsend, A., C. Öhlén, J. Bastin, H.G. Ljunggren, L. Foster, and K. Kärre. 1989. Association of class I major histocompatibility heavy and light chains induced by viral peptides. *Nature.* 340:443-448.
8. Ljunggren, H.G., N.J. Stam, C. Öhlén, J.J. Neefjes, P. Höglund, M.T. Heemels, J. Bastin, T.N. Schumacher, A. Townsend, and K. Kärre. 1990. Empty MHC class I molecules come out in the cold. *Nature.* 346:476-480.
9. Day, P.M., F. Esquivel, J. Lukszo, J.R. Bennink, and J.W. Yewdell. 1995. Effect of TAP on the generation and intracellular trafficking of peptide-receptive major histocompatibility complex class I molecules. *Immunity.* 2:137-147.
10. Öhlén, C., J. Bastin, H.G. Ljunggren, L. Foster, E. Wolpert, G. Klein, A.R. Townsend, and K. Kärre. 1990. Resistance to H-2-restricted but not to allo-H2-specific graft and cytotoxic T lymphocyte responses in lymphoma mutant. *J*. *Immunol.* 145:52-58.
11. Franksson, L., M. Petersson, R. Kiessling, and K. Kärre. 1993. Immunization against tumour and minor histocompatibility antigens by eluted cellular peptides loaded on antigen processing defective cells. *Eur. J. Immunol.* 23:2606-2613.
12. Aosai, F., C. Öhlén, H.G. Ljunggren, P. Höglund, L. Franksson, H. Ploegh, A. Townsend, K. Kärre, and H.J. Stauss. 1991. Different types of allospecific CTL clones identified by their ability to recognize peptide loading-defective target cells. *Eur. J. Immunol.* 21:2767-2774.
13. Rötzschke, O., K. Falk, S. Faath, and H.G. Rammensee. 1991. On the nature of peptides involved in T cell alloreactivity. *J. Exp. Med.* 174:1059-1071.
14. Wei, M.L, and P. Cresswell. 1992. HLA-A2 molecules in an antigen-processing mutant cell contain signal sequence-derived peptides. *Nature.* 356:443-446.
15. Henderson, R.A., A.L. Cox, K. Sakaguchi, E. Appella, J. Shabanowitz, D.F. Hunt, and V.H. Engelhard. 1993. Direct identification of an endogenous peptide recognized by multiple HLA-A2.1-specific cytotoxic T cells. *Proc. Natl. Acad. Sci. U.* S. A. 90:10275-10279.
16. Restifo, N.P., F. Esquivel, Y. Kawakami, J.W. Yewdell, J.J. Mule, S.A. Rosenberg, and J.R. Bennink. 1993. Identification of human cancers deficient in antigen processing. *J*. *Exp. Med.* 177:265-272.
17. Cromme, F.V., J. Airey, M.T. Heemels, H.L. Ploegh, P.J. Keating, P.L. Stern, C.J. Meijer, and J.M. Walboomers. 1994. Loss of transporter protein, encoded by the TAP-1 gene, is highly correlated with loss of HLA expression in cervical carcinomas. *J*. *Exp. Med.* 179:335-340.
18. Seliger, B., A. Hohne, A. Knuth, H. Bernhard, T. Meyer, R. Tampe, F. Momburg, and C. Huber. 1996. Analysis of the major histocompatibility complex class I antigen presentation machinery in normal and malignant renal cells: evidence for deficiencies associated with transformation and progression. *Cancer Res.* 56:1756-1760.
19. Garrido, F., T. Cabrera, A. Concha, S. Glew, F. Ruiz-Cabello, and P.L. Stern. 1993. Natural history of HLA expression during tumour development. *Immunol. Today.* 14:491-499.
20. Crooke, S.T., Bennett, C.F. 1996. Progress in antisense oligonucleotide therapeutics. *Annual Review of Pharmacology* & *Toxicology* 36:107-129.
21. Zon, G. 1995. Antisense phosphorothioate oligodeoxinucleotides: introductory concepts and possible mechanisms of toxicity. *Toxicology Letters,* Dec, 82-83:419-424.
22. Nair, S.K., D. Snyder, and E. Gilboa. 1996. Cells treated with TAP-2 antisense oligonucleotides are potent antigen-presenting cells in vitro and in vivo. *J*. *Immunol.* 156:1772-1780.
23. Sharma H.W., Narayanan, R. 1996. The NF-kappaB transcription factor in oncogenesis. *Anticancer Research,* Mar-Apr, 16(2):589-596.
24. Ohkawa, J., Koguma, T., Kohda, T., Taira, K. 1995. Ribozymes: from mechanistic studies to applications in vivo. *Journal of Biochemistry*, Aug, 118(2):251-258.
25. Putnam, D.A. 1996. Antisense strategies and therapeutic applications. *American Journal of Health-System Pharmacy,* Jan, 15;53(2):151-160.
26. Ulmer J.B., J.C. Sadoff and M.A. Liu. 1996. DNA vaccines. *Current Opinion in Immunology.* 8:531-536.
27. Tascon, RE., Colston MJ., Ragno, S., Stavropoulus, E., Gregory, D., Lowrie, DB. 1996. Vaccination against tuberculosis by DNA injection. *Nature Medicine,* Aug, 2(8):888-892.
28. Uebel S., T.H. Meyer, W Kraas, S. Kienle, G. Jung, K.H. Wiesmuller, and R. Tampe. 1995. Requirements for peptide binding to the human transporter associated with antigen processing revealed by peptide scans and complex libraries. *J*. *Biological Chemistry 270* (31):18512-18516.
29. WO 96/33272.
30. Strobl H., E. Riedl, C. Scheinecker, C. Bello-Fernandez, W.F. Pickl, K. Rappersberger, O. Majdic and W. Knapp. 1996. TGF-betal promotes in vitro development of dendritic cells from CD34⁺ hemopoetic progenitors. *J. Immunology*. 157(4):1499-507.
31. Kiertscher S.M., and Roth M.D. 1996. Human CD14⁺ leukocytes acquire the phenotype and function of antigen-presenting dendritic cells when cultured in GM-CSF and IL-4. *Journal of Leukocyte Biology* 59(2):208-218.
32. Obst, R., Armandola, EA., Nijenhuis, M., Momburg, F., Hammerling, GJ. 1995. TAP polymorphism does not influence transport of peptide variants in mice and humans. *European Journal of Immunology.* Aug. 25(8):2170-2176.
33. Hill, A., P. Jugovic, I. York, G. Russ, J. Bennink, J. Yewdell, H. Ploegh, and D. Johnson. 1995. Herpes simplex virus turns off the TAP to evade host immunity. *Nature.* 375:411-415.
34. Fruh, K., K. Ahn. H. Djaballah, P. Sempe, P.M. van Endert, R. Tampe, P.A. Peterson, and Y. Yang. 1995. A viral inhibitor of peptide transporters for antigen presentation. *Nature.* 375:415-418.
35. Rock, K.L., C. Graham, L. Rothstein, K. Clark, R. Stein, L. Dick, D. Hwang and A.L. Goldberg. 1994. Inhibitors of the proteasome block the degradation of most cell proteins and the generation of peptides presented on MHC class I molecules. *Cell.* 78:761-771.
36. Harding C.V., J. France, R. Song, J,M, Farah, S. Chatterjee, M. Iqbal, and R. Siman. 1995. Novel dipeptide aldehyds are proteasome inhibitors and block the MHC class I antigen processing pathway. *J*. *Immunol.* 155 :1767-1775.
37. Fehling H.J., W. Swat, C. Laplace, R. Kuhn, K. Rajewsky, U. Muller, and H. von Boehmer. 1992. MHC class I expression in mice lacking the proteasome subunit LMP-7. *Science* 265:1234.
38. Koller, B.H., P. Marrack, J.W. Kappler, and O. Smithies. 1990. Normal development of mice deficient in β₂-microglobulin, MHC class I proteins, and CD8⁺ T cells. *Science.* 248:1227-1230.
39. Van Kaer, L., P.G. Ashton-Rickardt, H.L. Ploegh, and S. Tonegawa. 1992. TAP1 mutant mice are deficient in antigen presentation, surface class I molecules, and CD4⁻8⁺ T cells. *Cell.* 71:1205-1214.
40. Ljunggren, H.G., L. Van Kaer, M.S. Sabatine, H. Auchincloss Jr, S. Tonegawa, and H.L. Ploegh. 1995. MHC class I expression and CD8⁺ T cell development in TAP1/β₂-microglobulin double mutant mice. *Int. Immunol.* 7:975-984.
41. Cerundolo, V., J. Alexander, K. Anderson, C. Lamb, P. Cresswell, A. McMichael, F. Gotch, and A. Townsend. 1990. Presentation of viral antigen controlled by a gene in the major histocompatibility complex. *Nature.* 345:449-452.
42. Lane, P., W. Gerhard, S. Hubele, A. Lanzavecchia, and F. McConnell. 1993. Expression and functional properties of mouse B7/BB 1 using a fusion protein between mouse CTLA4 and human γ1. *Immunology.* 80:56-61.
43. Inaba K., Inaba M., Romani N., Aya H., Deguchi M., Ikehara S., Murumatsu S. and Steinman R.M. 1992. Generation of large numbers of dendritic cells from mouse bone marrow cultures supplemented with granulocyte/macrophage colony-stimulating factor. *J*. *Exp. Med.* 176 (6):1693-1702.
44. Karre, K., H.G. Ljunggren, G. Piontek, and R. Kiessling. 1986. Selective rejection of H-2-deficient lymphoma variants suggests alternative immune defence strategy. *Nature.* 319:675-678.
45. Takebe, Y., Seiki, M., Fujisawa, J-I., Hoy, P., Yokota, K., Arai, K-I., Yoshida, M. and Arai, N. 1988. Srα Promotor: an efficient and versatile Mammalian cDNA expression system composed of the simina virus 40 early promoter and the R-U5 segment of human T-cell leukemia virus type 1 long terminal repeat. *Molecular and Cellular Biology,* 466-472.
46. Zhou, X., R. Glas, F. Momburg, G.J. Hammerling, M. Jondal, and H.G. Ljunggren. 1993. TAP2-defective RMA-S cells present Sendai virus antigen to cytotoxic T lymphocytes. *Eur. J. Immunol.* 23:1796-1801.
47. Wilson, J.B., Bell, J.L., Levine, A.J. 1996. Expression of Epstein-Barr virus nuclear antigen 1 induces B-cell neoplasia in transgenic mice. *EMBO Journal*, Jun 17, 15(12)3117-3126.
48. Dildrop R.MA.A., Zimmerman, K. Hsu, E., Tesfaye, A., DePinho, R., Alt, F.W. 1989. IgH enhancer-mediated deregulation of N-myc gene expression in transgenic mice: generation of lymphoid neoplasias that lack c-myc expression. *EMBO Journal.* Apr. 8 (4):1121-1128.

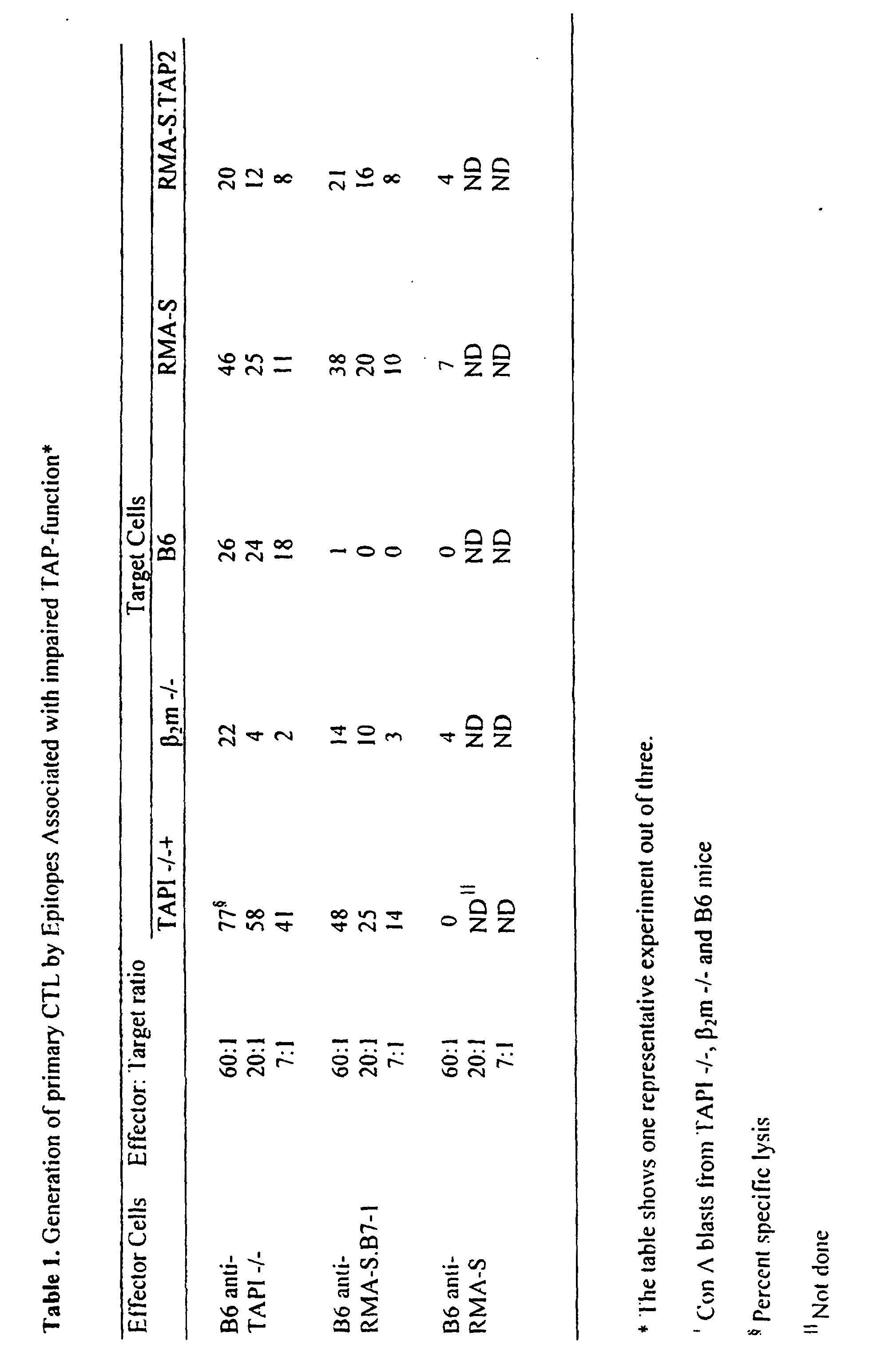

## Claims

1. Use of a substance that impairs cellular peptide processing for MHC presentation, the substance being **characterized in that** tumour cells treated with the substance are subject to specific lysis by CTL elicited by endogenous MHC class I dependent antigens of the TAP-deficient variant of said tumour cell which has been transfected with the stimulatory molecule B7-1, in the preparation of a pharmaceutical composition or vaccine for the treatment of cancer or a virus infection.

2. Use according to claim 1, wherein the substance is chosen from substances that inhibit the function and/or expression of TAP.

3. Use according to claim 2, wherein the substance is chosen from ICP47 of HSV type 1, IE 12 of HSV type 2, a gene encoding a TAP inhibitor, a gene encoding a nucleotide sequence that is complementary at least in part to the mRNA or DNA sequences encoding TAP, antisense oligonucleotides and RNA destroying ribozyme.

4. Use according to claim 1, wherein the substance inhibits the function and/or expression of the proteasome.

5. Use according to claim 4, wherein the substance is chosen from a peptide aldehyde Z-Leu-Leu-H, Lactacystin, a gene encoding a proteasome inhibitor, a nucleotide sequence that is complementary at least in part to the mRNA or DNA sequences encoding proteasome, antisense oligonucleotides and RNA destroying ribozyme.

6. In vitro method for providing cells which are capable of activating CD8⁺ T lymphocytes that selectively recognize cells showing impaired cellular peptide processing for MHC presentation, comprising the step of treating cells with an effective dose of a substance that impairs cellular peptide processing for MHC presentation, the substance being **characterized in that** tumour cells treated with the substance are subject to specific lysis by CTL elicited by endogenous MHC class I dependent antigens of the TAP-deficient variant of said tumour cell which has been transfected with the stimulatory molecule B7-1.

7. Method according to claim 6, wherein the substance is chosen from substances that inhibit the function and/or expression of TAP.

8. Method according to claim 7, wherein the substance is chosen from ICP47 of HSV type 1, IE 12 of HSV type 2, a gene encoding a TAP inhibitor, a gene encoding a nucleotide sequence that is complementary at least in part to the mRNA or DNA sequences encoding TAP, antisense oligonucleotides and RNA destroying ribozyme.

9. Method according to claim 6, wherein the substance inhibits the function and/or expression of the proteasome.

10. Method according to claim 9, wherein the substance is chosen from a peptide aldehyde Z-Leu-Leu-H, Lactacystin, a gene encoding a proteasome inhibitor, a nucleotide sequence that is complementary at least in part to the mRNA or DNA sequences encoding proteasome, antisense oligonucleotides and RNA destroying ribozyme.

11. Method according to any of claims 6 to 10, wherein the cells are autologous and/or hematopoetic cells.

12. Method according to claim 11, wherein the autologous and/or hematopoetic cells are dendritic cells or cells from cancer tissues.

13. In vitro method for providing immunological effector cells that selectively recognize cells showing impaired cellular peptide processing for MHC presentation, comprising the step of stimulating isolated immunological effector cells in vitro with cells provided according to the method of any of claims 6 to 12.

14. Method according to claim 13 wherein the immunological effector cells are CD8+ T lymphocytes.

15. Use of cells provided according to the method of any of claims 6 to 12, or antigens or epitopes expressed by such cells, in the preparation of a pharmaceutical composition or vaccine for the treatment of cancer or a virus infection.

16. Use of immunological effector cells provided according to the method of claims 13 or 14 in the preparation of a pharmaceutical composition or vaccine for the treatment of cancer or a virus infection.

17. Pharmaceutical composition or vaccine comprising a substance that impairs cellular peptide processing for MHC presentation, the substance being **characterized in that** tumour cells treated with the substance are subject to specific lysis by CTL elicited by endogenous MHC class I dependent antigens of the TAP-deficient variant of said tumour cell which has been transfected with the stimulatory molecule B7-1, additionally containing a pharmaceutically acceptable adjuvant selected from cytokines, genes for cytokines, costimulatory molecules, gold beads and/or liposomes and optionally pharmaceutically acceptable additives.

18. Pharmaceutical composition or vaccine comprising cells provided according to the method of any of claims 6 to 12, or antigens or epitopes expressed by such cells, and a pharmaceutically acceptable additive.

19. Pharmaceutical composition or vaccine comprising immunological effector cells provided according to the method of claim 13 or 14.

20. Kit comprising a substance that impairs cellular peptide processing for MHC presentation, the substance being **characterized in that** tumour cells treated with the substance are subject to specific lysis by CTL elicited by endogenous MHC class I dependent antigens of the TAP-deficient variant of said tumour cell which has been transfected with the stimulatory molecule B7-1, as a first component and cytokines, genes for cytokines, costimulatory molecules, gold beads and/or liposomes as a second component.

## Patentansprüche

1. Verwendung einer Substanz, die die zelluläre Peptidbearbeitung für die MHC-Präsentation beeinträchtigt, wobei die Substanz **dadurch gekennzeichnet ist, dass** Tumorzellen, die mit der Substanz behandelt werden, einer spezifischen Lysis durch CTL unterliegen, die durch endogene MHC-Klasse I-abhängige Antigene der TAP-defizienten Variante dieser Tumorzelle, die mit dem stimulatorischen Molekül B7-1 transfiziert worden ist, ausgelöst wird, in der Herstellung einer pharmazeutischen Zusammensetzung oder eines Vaccins für die Behandlung von Krebs oder einer Virusinfektion.

2. Verwendung gemäss Anspruch 1, wobei die Substanz ausgewählt wird aus Substanzen, die die Funktion und/oder Expression von TAP inhibieren.

3. Verwendung gemäss Anspruch 2, wobei die Substanz ausgewählt wird aus ICP47 vom HSV-Typ 1, IE 12 vom HSV-Typ 2, einem Gen, das einen TAP-Inhibitor codiert, einem Gen, das eine Nukleotidsequenz codiert, die zumindest teilweise zu den mRNA- oder DNA-Sequenzen, die TAP codieren, komplementär ist, Antisense-Oligonukleotiden und RNA-zerstörendem Ribozym.

4. Verwendung gemäss Anspruch 1, wobei die Substanz die Funktion und/oder Expression des Proteasoms inhibiert.

5. Verwendung gemäss Anspruch 4, wobei die Substanz ausgewählt wird aus einem Peptidaldehyd Z-Leu-Leu-H, Lactacystin, einem Gen, das einen Proteasom-Inhibitor codiert, einer Nukleotidsequenz, die zumindest teilweise zu den mRNA- oder DNA-Sequenzen, die das Proteasom codieren, komplementär ist, Antisense-Oligonukleotiden und RNA-zerstörendem Ribozym.

6. In vitro-Verfahren zur Bereitstellung von Zellen, die CD8⁺ T-Lymphozyten aktivieren können, die selektiv Zellen erkennen, die eine beeinträchtigte zelluläre Peptidbearbeitung für die MHC-Präsentation zeigen, umfassend den Schritt des Behandelns der Zellen mit einer wirksamen Dosis einer Substanz, die die zelluläre Peptidbearbeitung für die MHC-Präsentation beeinträchtigt, wobei die Substanz **dadurch gekennzeichnet ist, dass** Tumorzellen, die mit der Substanz behandelt werden, einer spezifischen Lysis durch CTL unterliegen, die durch endogene MHC-Klasse I-abhängige Antigene der TAP-defizienten Variante der Tumorzelle, die mit dem stimulatorischen Molekül B7-1 transfiziert worden ist, ausgelöst wird.

7. Verfahren gemäss Anspruch 6, wobei die Substanz ausgewählt wird aus Substanzen, die die Funktion und/oder Expression von TAP inhibieren.

8. Verfahren gemäss Anspruch 7, wobei die Substanz ausgewählt wird aus ICP47 vom HSV-Typ 1, IE 12 vom HSV-Typ 2, einem Gen, das einen TAP-Inhibitor codiert, einem Gen, das eine Nukleotidsequenz codiert, die zumindest teilweise zu den mRNA- oder DNA-Sequenzen, die TAP codieren, komplementär ist, Antisense-Oligonukleotiden und RNA-zerstörendem Ribozym.

9. Verfahren gemäss Anspruch 6, wobei die Substanz die Funktion und/oder Expression des Proteasoms inhibiert.

10. Verfahren gemäss Anspruch 9, wobei die Substanz ausgewählt wird aus einem Peptidaldehyd Z-Leu-Leu-H, Lactacystin, einem Gen, das einen Proteasom-Inhibitor codiert, einer Nukleotidsequenz, die zumindest teilweise zu den mRNA- oder DNA-Sequenzen, die das Proteasom codieren, komplementär ist, Antisense-Oligonukleotiden und RNA-zerstörendem Ribozym.

11. Verfahren gemäss einem der Ansprüche 6 bis 10, wobei die Zellen autologe und/oder hämatopoetische Zellen sind.

12. Verfahren gemäss Anspruch 11, wobei die autologen und/oder hämatopoetischen Zellen dendritische Zellen oder Zellen aus Krebsgewebe sind.

13. In vitro-Verfahren zur Bereitstellung von immunologischen Effektorzellen, die selektiv Zellen erkennen, die eine beeinträchtigte zelluläre Peptidbearbeitung für die MHC-Präsentation zeigen, umfassend den Schritt des Stimulierens von isolierten immunologischen Effektorzellen in vitro mit Zellen, die gemäss dem Verfahren gemäss einem der Ansprüche 6 bis 12 bereitgestellt werden.

14. Verfahren gemäss Anspruch 13, wobei die immunologischen Effektorzellen CD8⁺ T-Lymphozyten sind.

15. Verwendung von Zellen, bereitgestellt gemäss dem Verfahren gemäss einem der Ansprüche 6 bis 12, oder Antigene oder Epitope, die von solchen Zellen exprimiert werden, in der Herstellung einer pharmazeutischen Zusammensetzung oder eines Vaccins für die Behandlung von Krebs oder einer Virusinfektion.

16. Verwendung von immunologischen Effektorzellen, bereitgestellt gemäss dem Verfahren gemäss Anspruch 13 oder 14, in der Herstellung einer pharmazeutischen Zusammensetzung oder eines Vaccins für die Behandlung von Krebs oder einer Virusinfektion.

17. Pharmazeutische Zusammensetzung oder Vaccin, umfassend eine Substanz, die die zelluläre Peptidbearbeitung für die MHC-Präsentation beeinträchtigt, wobei die Substanz **dadurch gekennzeichnet ist, dass** Tumorzellen, die mit der Substanz behandelt werden, einer spezifischen Lysis durch CTL unterliegen, die durch endogene MHC-Klasse I-abhängige Antigene der TAP-defizienten Variante dieser Tumorzelle, die mit dem stimulatorischen Molekül B7-1 transfiziert worden ist, ausgelöst wird, zusätzlich enthaltend ein pharmazeutisch annehmbares Adjuvans, ausgewählt aus Cytokinen, Genen für Cytokine, costimulatorischen Molekülen, Goldbeads und/oder Liposomen und wahlweise pharmazeutisch annehmbare Additive.

18. Pharmazeutische Zusammensetzung oder Vaccin, umfassend Zellen, bereitgestellt gemäss dem Verfahren gemäss einem der Ansprüche 6 bis 12, oder Antigene oder Epitope, die von solchen Zellen exprimiert werden, und ein pharmazeutisch annehmbares Additiv.

19. Pharmazeutische Zusammensetzung oder Vaccin, umfassend immunologische Effektorzellen, bereitgestellt gemäss dem Verfahren von Anspruch 13 oder 14.

20. Kit, umfassend als eine erste Komponente eine Substanz, die die zelluläre Peptidbearbeitung für die MHC-Präsentation beeinträchtigt, wobei die Substanz **dadurch gekennzeichnet ist, dass** Tumorzellen, die mit der Substanz behandelt werden, einer spezifischen Lysis durch CTL unterliegen, die durch endogene MHC-Klasse I-abhängige Antigene der TAP-defizienten Variante dieser Tumorzelle, die mit dem stimulatorischen Molekül B7-1 transfiziert worden ist, ausgelöst wird, und Cytokine, Gene für Cytokine, costimulatorische Moleküle, Goldbeads und/oder Liposome als eine zweite Komponente.

## Revendications

1. Utilisation d'une substance qui altère le traitement peptidique cellulaire pour la présentation par le CMH (Complexe Majeur d'Histocompatibilité), la substance étant **caractérisée en ce que** des cellules tumorales traitées avec la substance sont soumises à une lyse spécifique par des lymphocytes T cytotoxiques (CTL) activés par des antigènes dépendants du CMH de classe I endogènes du variant déficient en TAP (transporteur associé au processus antigénique) de ladite cellule tumorale qui a été transfectée avec la molécule stimulatrice B7-1, pour la préparation d'une composition pharmaceutique ou d'un vaccin pour le traitement du cancer ou d'une infection virale.

2. Utilisation selon la revendication 1, dans laquelle la substance est choisie à partir de substances qui inhibent la fonction et/ou l'expression du TAP.

3. Utilisation selon la revendication 2, dans laquelle la substance est choisie à partir de la ICP47 du virus de l'herpès simplex (HSV)de type 1, de la IE 12 du HSV de type 2, d'un gène codant un inhibiteur du TAP, d'un gène codant une séquence nucléotidique qui est complémentaire au moins en partie des séquences d'ARNm ou d'ADN codant le TAP, des oligonucléotides anti-sens et d'un ribozyme détruisant l'ARN.

4. Utilisation selon la revendication 1, dans laquelle la substance inhibe la fonction et/ou l'expression du protéasome.

5. Utilisation selon la revendication 4, dans laquelle la substance est choisie à partir d'un peptide aldéhyde Z-Leu-Leu-H, de lactacystine, d'un gène codant un inhibiteur de protéasome, d'une séquence nucléotidique qui est complémentaire au moins en partie des séquences d'ARNm ou d'ADN codant un protéasome, des oligonucléotides anti-sens et d'un ribozyme détruisant l'ARN.

6. Procédé in vitro pour fournir des cellules qui sont capables d'activer des lymphocytes T CD8⁺ qui reconnaissent sélectivement les cellules présentant un traitement peptidique cellulaire altéré pour la présentation par le CMH, comprenant l'étape de traiter les cellules avec une dose efficace d'une substance qui altère le traitement peptidique cellulaire pour la présentation par le CMH, la substance étant **caractérisée en ce que** les cellules tumorales traitées avec la substance sont soumises à une lyse spécifique par des CTL activés par des antigènes dépendants du CMH de classe I endogènes du variant déficient en TAP de ladite cellule tumorale qui a été transfectée avec la molécule stimulatrice B7-1.

7. Procédé selon la revendication 6, dans lequel la substance est choisie à partir de substances qui inhibent la fonction et/ou l'expression du TAP.

8. Procédé selon la revendication 7, dans lequel la substance est choisie à partir d'ICP47 du HSV de type 1, d'IE 12 du HSV de type 2, d'un gène codant un inhibiteur du TAP, d'un gène codant une séquence nucléotidique qui est complémentaire au moins en partie des séquences d'ARNm ou d'ADN codant le TAP, des oligonucléotides anti-sens et d'un ribozyme détruisant l'ARN.

9. Procédé selon la revendication 6, dans lequel la substance inhibe la fonction et/ou l'expression du protéasome.

10. Procédé selon la revendication 9, dans lequel la substance est choisie à partir d'un peptide aldéhyde Z-Leu-Leu-H, de lactacystine, d'un gène codant un inhibiteur du protéasome, d'une séquence nucléotidique qui est complémentaire au moins en partie des séquences d'ARNm ou d'ADN codant un protéasome, des oligonucléotides anti-sens et d'un ribozyme détruisant l'ARN.

11. Procédé selon l'une quelconque des revendications 6 à 10, dans lequel les cellules sont des cellules autologues et/ou hématopoïétiques.

12. Procédé selon la revendication 11, dans lequel les cellules autologues et/ou hématopoïétiques sont des cellules dendritiques ou des cellules provenant de tissus cancéreux.

13. Procédé in vitro pour fournir des cellules effectrices immunologiques qui reconnaissent sélectivement les cellules présentant un traitement peptidique cellulaire altéré pour la présentation par le CMH, comprenant l'étape de stimuler des cellules effectrices immunologiques isolées in vitro avec des cellules fournies selon le procédé de l'une quelconque des revendications 6 à 12.

14. Procédé selon la revendication 13, dans lequel les cellules effectrices immunologiques sont des lymphocytes T CD8⁺.

15. Utilisation de cellules fournies selon le procédé de l'une quelconque des revendications 6 à 12, ou des antigènes ou épitopes exprimés par de telles cellules, pour la préparation d'une composition pharmaceutique ou d'un vaccin destiné au traitement du cancer ou d'une infection virale.

16. Utilisation de cellules effectrices immunologiques fournies selon le procédé de la revendication 13 ou 14 pour la préparation dune composition pharmaceutique ou d'un vaccin destiné au traitement du cancer ou d'une infection virale.

17. Composition pharmaceutique ou vaccin comprenant une substance qui altère le traitement peptidique cellulaire pour la présentation par le CMH, la substance étant **caractérisée en ce que** les cellules tumorales traitées avec la substance sont soumises à une lyse spécifique par des CTL activés par des antigènes dépendants du CMH de classe I endogènes du variant déficient en TAP de ladite cellule tumorale qui a été transfectée avec la molécule stimulatrice B7-1, contenant de plus un adjuvant pharmaceutiquement acceptable, choisi à partir de cytokines, de gènes pour les cytokines, de molécules costimulatrices, de billes en or et/ou de liposomes et, facultativement, d'additifs pharmaceutiquement acceptables.

18. Composition pharmaceutique ou vaccin comprenant des cellules fournies selon le procédé de l'une quelconque des revendications 6 à 12, ou d'antigènes ou d'épitopes exprimés par de telles cellules, et un additif pharmaceutiquement acceptable.

19. Composition pharmaceutique ou vaccin comprenant des cellules effectrices immunologiques fournies selon le procédé de la revendication 13 ou 14.

20. Kit comprenant une substance qui altère le traitement peptidique cellulaire pour la présentation par le CMH, la substance étant **caractérisée en ce que** les cellules tumorales traitées avec la substance sont soumises à une lyse spécifique par des CTL activés par des antigènes dépendants du CMH de classe I endogènes du variant déficient en TAP de ladite cellule tumorale qui a été transfectée avec la molécule stimulatrice B7-1, en tant que premier composant et de cytokines, gènes pour les cytokines, molécules costimulatrices, billes en or et/ou liposomes en tant que second composant.
